# EUROPEAN PATENT SPECIFICATION

(11) **EP 1 750 522 B1**
(45) Date of publication and mention of the grant of the patent: **20.04.2011**
(21) Application number: 05754077.5
(22) Date of filing: 26.05.2005
(51) Int. Cl.: A23K 1/16

(54) **USE OF PET FOOD COMPOSITIONS**
VERWENDUNG VON HEIMTIERFUTTERZUSAMMENSETZUNGEN
UTILISATION DE COMPOSITIONS DE RATIONS ANIMALES

(30) Priority: 27.05.2004 US 855080
(43) Date of publication of application: 14.02.2007
(73) Proprietor: THE IAMS COMPANY, Dayton, Ohio 45414 (US)
(72) Inventor: KELLEY, Russell, Lee, Eaton, Ohio 45320 (US); LEPINE, Allan, John, Dayton, Ohio 45414 (US); WATKINS, Bruce, A., West Lafayette, Indiana 47906 (US)
(74) Representative: Goodier, Claire-Louise
(86) International application number: PCT/US2005/018702
(87) International publication number: WO 2005/117603

(56) References cited:
- WO-A-01/60356
- WO-A-01/82720
- WO-A-98/46764
- WO-A-02/092073
- WO-A-03/075670

## Description

### FIELD OF THE INVENTION

The present invention is directed to pet food compositions comprising stearidonic acid.

### BACKGROUND OF THE INVENTION

Dietary fat can influence bone metabolism by altering prostaglandin biosynthesis IGF actions, as well as gene and protein expression. Prostaglandins, produced locally from 20-carbon essential fatty acid precursors (arachidonic acid and eicosapentaenoic acid) in osteogenic cells, regulate both bone formation and bone resorption. In support of the relationship between dietary polyunsaturated fatty acids, prostaglandins and bone metabolism, it has been reported that dietary lipids modulate ex *vivo* bone PGE₂ production and the concentrations of IGF-I in bone tissues, and lead to altered bone formation rates in growing chicks and rats. In these experiments, animals provided with long-chain n-3 fatty acids demonstrated an increased rate of bone formation suggestive of a stimulatory effect on osteoblastic activity. The favorable effect of n-3 fatty acids on bone modeling in growing animals was supported by the observation of reduced bone mineral loss in ovariectomized rats supplemented with eicosapentaenoic acid (often referred to as "20:5n-3" or "EPA") and docosahexaenoic acid (often referred to as "22:6n-3" or "DHA"). See Watkins et al., "Omega-3 polyunsaturated fatty acids and skeletal health," P.S.E.B.M., Vol. 226(6), pp. 485-497 (2001). These investigations indicate that a bone sparing effect of long-chain n-3 fatty acids may be associated with diminished bone resorption and increased bone formation. The responses observed in bone tissue indicate that moderating the action of n-6 fatty acids (*e.g*., linoleic acid) with long-chain n-3 fatty acids or CLA can benefit bone remodeling.

Studies in dogs, chicks, sheep, and rats have shown that dietary lipids transform the fatty acid composition of bone compartments and can impact the local production of agents (*e.g.,* prostaglandins) that influence bone modeling/remodeling. It has been further reported that dietary n-3 polyunsaturated fatty acid (PUFA) lowered the concentration of arachidonic acid (often referred to as "20:4n-6" or "AA") in bone and cartilage tissues, and depressed *ex vivo* PGE₂ production in bone organ cultures. See Watkins et al., "Bioactive fatty acids: Role in bone biology and bone cell function, "Prog. Lipid Res, Vol. 40, pp. 125-148 (2001). In another rat study, *ex vivo* PGE₂ production in bone organ culture was significantly reduced in rats given diets with a lowest dietary ratio of n-6/n-3 fatty acids (n-6/n-3 = 1,2 ~ 2.6) compared with those on diets with a higher dietary ratio (n-6/n-3 = 10 ~ 24).

Regression analysis revealed a significant positive correlation between *ex vivo* production of PGF₂ and the ratio of AA/EPA in bone but a significant negative correlation between bone formation rate and either the ratio of AA/EPA or PGE₂ in bone. Moreover, the activity of serum bone-specific alkaline phosphatase was greater in rats given a diet high in n-3 fatty acids that further support the positive action of these fatty acids on bone formation. These results demonstrated that the dietary ratio of n-6/n-3 fatty acids modulates bane PGE₂ production and the activity of serum bone-specific alkaline phosphatase in growing rats.

WO 03/015670 refers to methods for treating and preventing disorders associated with elevated TNF-α and/or IL-Iβ such as inflammatory disorders by administering stearidonic acid. WO 98/46764 discloses compositions and methods for preparing polyunsaturated fatty acids including stearidonic acid, in plants or in parts thereof. The polyunsaturated fatty acids are useful for the treatment of various pathological states, including inflammation and cancer. WO 01/60356 discloses a method for improving bone modeling and chondrocyte functioning in growing canines.

Despite these advances, there remains a need in the art to further understand the fatty acids which are implicated in the promotion of bone health and joint health, including control of inflammation and related benefits.

### SUMMARY OF THE INVENTION

The present invention is directed to compositions which utilize stearidonic acid (SDA), which is an omega-3- fatty acid.

The present invention is directed to the use of a pet food composition comprising stearidonic acid for promoting chondrocyte functioning, promoting and/or maintaining tissue concentration of omega-3-fatty acid and combinations thereof.

### DETAILED DESCRIPTION OF THE INVENTION

All percentages and ratios are calculated by weight unless otherwise indicated. All percentages and ratios are calculated based on the total composition unless otherwise indicated.

The compositions herein may comprise, consist essentially of, or consist of any of the elements as described herein.

As used herein, the term "pet" means a domestic dog or cat.

As used herein, the term "geriatric animal" or the like is a pet which is considered middle-aged or older in accordance with standards commonly utilized in the art, with the following proviso: a geriatric dog is a domestic dog at the age of about 5 or above; and a geriatric cat is a domestic cat at the age of about 6 or above.

As used herein, the term "large breed dog" means a domestic dog which is, or is estimated to be, about 14 kg (30 pounds) of weight or greater upon reaching the age of 3 years old. Optionally, the term "large breed dog" means a domestic dog which is, or is estimated to be, about 18 kg (40 pounds) of weight or greater upon reaching the age of 3 years old. Thus, large breed dogs will include dogs less than the age of 3, provided such dogs are estimated to reach the defined weight upon reaching the age of 3 years old. Non-limiting examples of large breed dogs may include those in the sporting group, hound group, working group, hound group, herding group, or miscellaneous classes as described by the American Kennel Club.

As used herein, the term "kitten" refers to a domestic cat which is about 3 years old or less, alternately about 2 years old or less, alternately about 1 year old or less.

As used herein, the term "puppy" refers to a domestic dog which is about 3 years old or less, alternately about 2 years old or less, alternately about 1 year old or less,

### Compositions of the Present Invention

The present compositions are useful for a variety of purposes. White such methods are intended for domestic dogs and cats, the methods may be particularly useful for geriatric animals, large breed dogs, or growing puppies or kittens.

The present inventive compositions are pet food compositions comprising stearidonic acid (often referenced herein as "SDA"). SDA is an omega-3-fatty acid. As is well-understood in the art, omega-3-fatty acids are those fatty acid materials having an omega-3 double bond wherein the first double bond in the carbon chain is positioned between the third and fourth carbon atoms of the fatty acid chain, when counting from the omega (distal) methyl carbon atom of the chain. SDA is an 18-carbon, omega-3-fatty acid, which may be referred to in the art as 6Z, 9Z, 12Z, 15Z-octaedecatetraenoic acid or moroctic acid.

All forms of SDA are contemplated herein. For example, SDA may be provided as a free fatty acid or as a triglyceride. As such, wherein SDA or any other fatty acid is mentioned herein, such fatty acid include the free form of the fatty acid as well as other forms such as ethyl (or other) esters or the naturally occurring triglyceride or other form. The terms SDA or other specific terms are utilized for convenience as will be commonly understood in the art to include all forms of such termed material.

In certain embodiments of the invention, the compositions comprise a concentrated source of SDA in order to provide all or a portion of the SDA present in the composition. As used herein, the term "concentrated source of stearidonic acid," "concentrated source of SDA," or the like refers to a component which is an additive to the composition which comprises at least 0,1% of SDA, by weight of the concentrated source. Optionally, the concentrated source comprises at least 0.4%, or at least 0.7%, or at least 1% of the SDA, by weight of the concentrated source.

Readily available concentrated source include, for example, nuls, seeds, extracts thereof (for example, a seed oil), transformed plant oils and oilseeds, various fish oils, or mixtures thereof. In certain embodiments herein, the concentrated source may be selected from plant families selected from the group consisting of *Cannabis sativa* (for example, comprising from 0.3:% to 2% of SDA), *Ribes nigium* (for example, comprising from 2% to 4% of SIDA), *Echium: plantagineum* (for example, comprising from 12% to 14% of SDA), or *Echium vulgaris* (for example, comprising from 18% to 20% of SDA). Illustrative concentrated sources herein include those selected from the group consisting of black currant seed oil, hemp seed oil echium oil, and mixtures thereof (inclusive of all varieties). Echium oil is commercially available from Croda International PLC of Great Britain, and other illustrative concentrated sources are widely available from a variety of commercial sources.

In alternative or additional embodiments herein, the pet food composition comprises an amount of SDA effective to provide a function selected from promotion of chondrocyte function, maintenance ot tissue concentration of omega-3-fatty acids, promotion of tissue concentration of omega-3-fatty acids, and combinations thereof in a pet following administration (preferably oral administration) to the pet. These functions are further described herein below with respect to *Methods of the Present. Invention*. As used herein, the term "effective amount," with reference to the SDA used herein, means that amount of SDA sufficient to provide the referenced benefit. The specific "effective amount" will vary with such factors as the physical condition of the pet, physiological state, age, gender, breed, duration of treatment, the nature of concurrent therapy (if any), the specific form of composition to be used, or the like, which will be well understood by one ordinary skill given the disclosures provided herein.

In alternative of additional embodiments herein, the pet food composition may comprise at least 0,001% of SDA, alternatively at least 0.01% SDA, alternatively at least 0.1% of SDA, all by weight of the composition, Still further, the pet food composition may comprise from 0,001% to 75% alternatively from 0.001% to 5%, alternatively from 0.001% to 1%, or alternatively from 0.01% to 0.5% of SDA, all by weight of the composition. Pet food compositions; which are supplements may tend to have higher levets of SDA relative to nutritionally balanced pet food compositions intended for daily feed. Even Further, supplement forms such as tablets, capsules, or the like may often for example: comprise higher levels of SDA relative to biscuits, chews, gravies or other like treats, including milks or milk replacers, or relative to pet food compositions intended for daily feed. The ordinarily skilled artisan will be able to make the appropriate determination.

In still further alternative or additional embodiments herein, the pet food composition comprises a level of SDA based on the weight of the total poylyunsaturated fatty acids of the composition; for example, in one embodiment, the composition may comprise from 0.1% to 5% of SDA, by weight of the total polyunsaturated fatty acids of the composition. Polyunsaturated fatty acids are well-understood in the art, and will include, but may not be limited to, omega-3 fatty acids and omega-6-fatty acids.

The pet food composition may be of any form, preferably forum which is orally administrable. For example, the composition may be a nutritionally balanced pet food composition or a supplement. As used herein, the term "nutritionally balanced," with reference to a pet food composition, means that the composition has known required nutrients to sustain life in proper amounts and proportion based on recommendations of recognized authorities in the field of pet nutrition. Nutritionally balanced pet food compositions are readily understood in the art, for examples, dry foods, semi-moist foods, and wet foods, all utilized as pet daily foods. Supplements may include dosage forms such as tablets, capsules, or the like, or other forms such as biscuits, chews, gravies (or other toppers), yogurts, milk replacers, or other forms. While supplements are not typically nutritionally balanced, it is certainly acceptable if such supplements are nutritionally balanced.

Other components are beneficial for inclusion in the composition used herein, but are optional for purposes of the invention. For example, pet food compositions intended for daily freed are preferably nutritionally balanced. In one embodiment, such pet food compositions may comprise, on a dry matter basis, from 20% to 50% protein, or from 22% to 40% protein, by weight of the pet food composition. As another example, the pet food compositions may comprise, on a dry matter basis, from 5% to 35% fat, or from 10% to 30% fat, by weight of the pet food composition. In another embodiment, supplement compositions such as biscuits, chews, and other such forms may comprise, on a dry matter basis, from 20% to 50% protein, or from 22% to 40% protein, by weight of the supplement composition. As another example, these types of supplement compositions may comprise, on a dry matter basis, from 5% to 35% fat, or from 10% to about 30% fat, by weight of the supplement compositions. As yet another example, supplement compositions such as graves or other toppers may often comprise from about at least 0.5% protein, or at least 0.8% protein, by weight of the supplement composition. As yet another example, supplement compositions such as gravies of other toppers may often comprise from about at least 1%, fat, or at least 2% fat, or from 1% to 5% fat, by weight of the supplement composition. As yet another example, supplement compositions such as gravies or other toppers may often comprise from about at least about 50% moisture, or at least 70% moisture, or from 70% to 99% moisture. Daily feed and supplement compositions intended for use by pets are, of course, commonly known in the art.

### Methods of the Present Invention

The methods of the present invention comprise administering, preferably orally administering (*i.e.*, through ingestion) a composition of the present invention to a pet to provide one or more health benefits described herein. In particular, the present methods are those selected from the group consisting of promoting chondrocyte functioning maintaining tissue concentration of omega-3-fatty acids, promoting tissue concentration of omega-3-fatty acids, and combinations thereof, including the like, in a pet comprising administration of a composition comprising SDA to the pet. While such methods are intended for domestic dogs and cats, the methods may be particularly useful for geriatric, animals, large breed dogs, or growing puppies or kittens. The compositions comprising the SDA may be any of a variety of compositions described herein above.

As has been surprisingly discovered herein, the provision of SDA to the pet results in maintenance or promotion of omega-3-fatty acid tissue concentrations. As such, in one embodiment, the present methods are directed to this discovery. Omega-3-fatty acids are commonly known in the art, and will include docosahexaenoic acid (commonly referred to as "DHA") and eicosapentaenoic acid (commonly referred to as "EPA").

Alternatively of additionally, the invention is directed to methods of promoting chondrocyte functioning. As is well-khown in the art, growth cartilage in long bones contain chondrocytes which initiate bone mineralization through matrix vesicles which have been described as lipid-enclosed microenvironments containing acidic phospholipids that exhibit a high affinity for binding calcium ions.

As used herein, with respect to the methods of the present invention, the terms "administering," "administration" or the like means that the referenced pet is provided one or more compositions herein; such provision may be acute or systemic. As used herein with respect to the processes of this invention, the terms "orally administering," "oral administration" or the like means that the referenced pet ingests or is directed to ingest (interpreted broadly as including mere provision of the composition to the pet) one or more compositions described herein, or the owner or guardian of such animal is directed to provide one or more compositions to the pet. Wherein the owner is directed to provide, such direction may be that which instructs and/or informs the owner that use of the composition may and/or will provide one or more of the benefits described herein, such as promotion of chondrocyte functioning (including cartilage health), maintaining tissue concentration of omega-3-fatty acids, promoting tissue concentration of omega-3-fatty acids, combinations thereof, including the like. For example, such direction may be oral direction (*e.g*., through oral instruction from, for example, a veterinarian, other health professional, sales professional or organization, and/or radio or television media (*i.e*., advertisement) or written direction (*e.g*., through written direction from, for example, a veterinarian or other health professional (*e.g*., scripts), sales professional or organization (*e.g*., through, for example, marketing brochures, pamphlets, or other instructive paraphernalia), written media (*e.g*., internet, electronic mail, or other computer-related media), and/or containing devices associated with the composition (*e.g*., a label present on a package containing the composition).

The compositions may be administered in accordance with a variety of frequencies or durations. For example, wherein the compositions are food compositions intended for daily feed, the compositions are typically administered from once daily to about four times daily, alternately from once daily to about three times daily, alternately from once daily to about two times daily, alternately *ad libitum.* However, daily administration is not necessary for any of the pet food compositions herein. For example, nutritionally balanced pet food compositions of the present invention may be interchanged with nutritionally balanced food compositions not in accordance with the present invention (for example, provision of both on a predetermined or rotating schedule). Supplements may or may not be provided daily.

In order to achieve the benefits herein, it is preferred that the compositions are administered for at least one week, alternatively at least two weeks, alternatively at least three weeks, alternately at least four weeks, alternatively at least 6 weeks, alternately at least eight weeks, or in an unlimited duration.

### Examples

The following examples are provided to illustrate the invention and BTC not intended to limit the scope thereof in any manner. The examples describe experimental methodology which may be employed to determine the benefit of SDA according to the claims.

Experiments are conducted to determine how SDA treatment of osteoblast-like cells in culture influence the polyunsaturated fatty acid (PUFA) composition. The osteoblast cell line MC3T3-E1 is cultured in growth medium (GM) consisting of DMEM supplemented with 10% fetal bovine serum (FBS), ascorbic acid, β-glycerol-phosphate, and 1% antimicrobial solution. The cells are plated in 24-well plates at a density of 1.2 x 10⁴ cells per ml for 3 drays until confluent. Once confluent corresponding to the initialization of the osteoblastic phenotype, the medium is either changed for SDA or linoleic acid (LNA) treatment or cultured further in GM until subjected to the various treatments. At the end of the 3 day fatty acid treatment period, cultures arc exposed to agents that induce COX-2 expression. The two agents utilized are forskolin (FSK) and interleukin-1 (IL-1). These agents are selected on the basis of their respective actions on the COX-2 gene. Forskolin, an activator of adenylate cyclase, increases cyclic-AMP (cAMP) concentrations. Interleukin-1, itself a bone resorption agent binding to its receptor on the cellular membrane, launches a cascade resulting in the activation of the transcription factor nuclear-factor κ-B (NFκB). Cyclic-AMP and NFκB both activate the expression of the COX-2 gene, Cyclic-AMP activates the expression of the COX-2 gene via the cAMP-response element binding protein (GREBP), which binds to the cAMP response element (CRE) in the COX-2 promoter. The COX-2 promoter also contains an NFκB binding element. Thus, these two compounds induce COX-2 and increase levels of the COX-2 protein through gene activation, albeit via two different pathways, offering a unique opportunity to demonstrate the interaction of nutraceutical fatty acids with the signaling pathways pertinent to COX-2 expression.

The following are determined as outcome measures:
Fatty Acid Compositional Analysis of Osteoblasts. Osteoblast cells exposed to the PUFA treatments (SDA and LNA) are subjected to a gas chromotographic analysis of the FAME from extracted lipids of washed cells.
MTS Assay. To determine if the fatty acid treatments are growth inhibitory to osteoblast cells, an assay employing a tetrazolium compound, 3-(4,5-dimethylthiazol-2-yl)-5-(3-carboxymethoxyphenyl)-2-(4-sulphenyl)-2H-tetrazolium, inner salt; also known as MTS (Cell Titer 96 Aqueous, Promega, Madison, WI) is utilized. MTS is converted to a colored product by living cells. Absorption is readily measured by spectrometry, and it is directly related to the number of viable cells. The results of this assay are comparable to those obtained using a ³H-thymidine incorporation assay. The osteoblast cells, MC3T3-E1, are cultured in 96-well plates to confluence. At confluence, medium was changed to GM supplemented with various concentrations of the fatty acids for 3 days. After 3 days, the MTS solution is added directly to the wells, the plate incubated at 37°C for 60 min and then A₄₉₀ read using a microplate reader (Molecular Dynamics, Amersham Biosciences Corporation, Piscataway, NJ).
Cis-parinaric Acid Assay. As a consequence of their structures, PUFA are susceptible to oxidation and the subsequent formation of free radical compounds, which can be genotoxic. Therefore experiments were performed to examine if PUFA enrichment treatments influence oxidative damage downstream. One unique method to determine susceptibility to oxidative damage is through the use of cis-parinaric acid [cPnA, conjugated tetraene 18:4 (9,11,13,15)], a fatty acid that naturally fluoresces when incorporated into cellular membranes and is used to test lipid oxidation. In these experiments, cells are cultured to confluence and the medium is changed to GM supplemented with PUFA for 24 hr, after which 20µM cPnA is added directly to the medium from 2 to 4 hr to allow esterification into membrane phospholipids. Following cPnA loading, the cells are washed 2x in phosphate-buffered saline (PBS). After washing, 100µM hydrogen peroxide (H₂O₂, an inducer of oxidative stress) is added in PBS and the cPnA fluorescence is measured for 30 min using a fluorescent microplate reader.
Western Blot. Analysis of COX and PLA₂ proteins are performed on cell cultures after confluence. The induction method is varied to assess the influence of FBS (which can induce COX-2 expression, induction time, inducer type, and inducer concentration) on the expression of COX-1, COX-2, and cytoplasmic phospholipase A₂ (cPLA₂) protein. Optical density is determined using Optimus image analyzing software (version 6.1). Optical density values for COX-1, COX-2, and cPLA₂ protein bands are normalized against those obtained for actin.
COX Gene Expression. The levels of COX gene expression are evaluated a by reverse-transcription polymerase chain reaction (RT-PCR) to amplify total RNA. Total cellular RNA is isolated with a kit (Ambion, Austin, TX) and pooled from 3 identical wells from a 24-well plate. 100 ng of total RNA and 100 ng of each gene specific primer for COX-1, COX-2, and actin are added to a Ready-To-Go RT-PCR bead (Amersham Biosciences Corporation, Piscataway, NJ) following the manufacturer's instructions. Levels of COX-1 and COX-2 mRNA are normalized to actin expression and UN SCAN-IT software (Version 5.1, Silk Scientific Inc., Orem, UT) is used in the analysis of the PCR bands, which are resolved through a 3% agarose gel and stained with ethidium bromide.
PGE₂ Formation. In the experiments, already detailed in the section on Western blotting, PGE₂ levels in the medium of induced and non-induced cells are determined using an enzyme immunoassay kit (Cayman Chemical, Ann Arbor, MI).
Enzyme activity: Alkaline phosphatase (ALP) activity is determined by the method of Lowry.
Collagen Synthesis. Collagen synthesis is another commonly used marker for osteoblastic bone forming activity. Collagen production peaks during the matrix maturation stage. Osteoblast cells cultured in fatty acid-supplemented GM for 5days once confluent (the longer fatty acid treatment time allows for the accumulation of assembly of collagen) are used in the experiments.

Results of the foregoing non-limiting examples indicate that the addition of SDA at increasing treatment levels results in increased concentrations of total omega-3 fatty acid in osteoblasts. These results demonstrate that SDA particularly elevates EPA and 22:5n-3, which are two fatty acids that can directly or indirectly modify prostanoid synthesis in bone tissues and osteoblasts. In other cell cultures, SDA is more effective in altering the concentrations of omega-3 fatty acid than linolenic acid (18:3n-3). Moreover, SDA is a fatty acid that has biological activity to attenuate PGE₂ production in osteoblasts. Still further, SDA results in the highest activity of alkaline phosphatase for all time points evaluated, relative to linolenic acid.

Experiments are also designed to evaluate the effect of SDA on the pet, such as a dog or cat. As an example, beagles are provided with a low essential fatty acid diet for a period of 90 days. Diets supplemented with SDA or linolenic acid are provided for the subsequent 30 days. Results indicate elevated concentrations of EPA and 22:5n-3 in red blood cell membranes, plasma, and liver tissue in dogs which are fed the diets supplemented with SDA as compared to those dogs fed diets supplemented with linolenic acid. Likewise, a trend of decreased arachidonic acid in plasma and liver of dogs which are fed the diets supplemented with SDA. Without intending to be limited by theory, these results indicate that dietary SDA results in increased tissue concentrations of key omega-3 fatty acids to a greater extent relative to linolenic acid by entering the metabolic pathway subsequent to delta-6-desaturase enzyme. These increases in omega-3 fatty acids and the trend of decreased arachidonic acid in the dog represent changes suggesting a significant health benefit for the pet and reflect an advantage of using SDA as a dietary component in pet food compositions over existing sources of omega-3 fatty acids.

## Claims

1. Use of a pet food composition comprising stearidonic acid for promoting chondrocyte functioning, promoting and/or maintaining tissue concentration of omega-3-fatty acids and combinations thereof.

2. Use of the pet food composition according to claim 1 comprising a concentrated source of stearidonic acid.

3. Use of the pet food composition according to claim 2, wherein the concentrated source is selected from the group consisting of seeds, nuts, extracts thereof, and mixtures thereof.

4. Use of the pet food composition according to any of claims 2 or 3, wherein the concentrated source is selected from the group consisting of black currant seed oil, hemp seed oil, echium oil, and mixtures thereof.

5. Use of the pet food composition according to any of claims 2 to 4, wherein the concentrated source comprises at least 0,4% of stearidonic acid, by weight of the concentrated source;

6. Use of the pet food composition according to any of the preceding claims comprising at least 0,01% of stearidonic acid, preferably at least 0,1% of stearidonic acid, by weight of the composition.

7. Use of the pet food composition according to any of the preceding claims which is a nutritionally balanced pet food composition.

8. Use of the pet food composition according to any of claims 1 to 6 which is a supplement.

## Patentansprüche

1. Verwendung einer Tiemahrungszusammensetzung, umfassend Stearidonsäure zum Verbessern der Chondrozytenfunktion, Verbessern und/oder Beibehalten der Gewebekonzentration von Omega-3-Fettsäuren und Kombinationen davon.

2. Verwendung der Tiernahrungszusammensetzung nach Anspruch 1, umfassend eine konzentrierte Quelle von Stearidonsäure.

3. Verwendung der Tiernahrungszusammensetzung nach Anspruch 2, wobei die konzentrierte Quelle ausgewählt ist aus der Gruppe bestehend aus Samen, Nüssen, Extrakten davon und Mischungen davon.

4. Verwendung der Tiernahrungszusammensetzung nach einem der Ansprüche 2 oder 3, wobei die konzentrierte Quelle ausgewählt ist aus der Gruppe bestehend aus Schwarze-Johannisbeere-Samenöl, Hanfsamenöl, Echium-Öl und Mischungen davon.

5. Verwendung der Tiernahrungszusammensetzung nach einem der Ansprüche 2 bis 4, wobei die konzentrierte Quelle mindestens 0,4 Gew.-% der konzentrierten Quelle Stearidonsäure umfasst;

6. Verwendung der Tiernahrungszusammensetzung nach einem der vorstehenden Ansprüche, umfassend mindestens 0,01 Gew.-% Stearidonsäure, vorzugsweise mindestens 0,1 Gew.-% der Zusammensetzung Stearidonsäure.

7. Verwendung der Tiernahrungszusammensetzung nach einem der vorstehenden Ansprüche, die eine ernährungsmäßig ausgeglichene Tiernahrungszusammensetzung ist.

8. Verwendung der Tiernahrungszusammensetzung nach einem der Ansprüche 1 bis 6, die ein Ergänzungsmittel ist.

## Revendications

1. Utilisation d'une composition alimentaire pour animaux de compagnie comprenant de l'acide stéaridonique pour favoriser le fonctionnement des chondrocytes, favoriser et/ou maintenir la concentration tissulaire en acides gras oméga-3 et leurs combinaisons.

2. Utilisation de la composition alimentaire pour animaux de compagnie selon la revendication 1, comprenant une source concentrée d'acide stéaridonique.

3. Utilisation de la composition alimentaire pour animaux de compagnie selon la revendication 2, dans laquelle la source concentrée est choisie dans le groupe constitué de graines, fruits à coques, extraits de ceux-ci, et leurs mélanges.

4. Utilisation de la composition alimentaire pour animaux de compagnie selon l'une quelconque des revendications 2 ou 3, dans laquelle la source concentrée est choisie dans le groupe constitué d'huile de graines de groseilles noires, huile de graines de chanvre, huile de vipérine, et leurs mélanges.

5. Utilisation de la composition alimentaire pour animaux de compagnie selon l'une quelconque des revendications 2 à 4, dans laquelle la source concentrée comprend au moins 0,4 % d'acide stéaridonique, en poids de la source concentrée ;

6. Utilisation de la composition alimentaire pour animaux de compagnie selon l'une quelconque des revendications précédentes comprenant au moins 0,01 % d'acide stéaridonique, de préférence au moins 0,1 % d'acide stéaridonique, en poids de la composition.

7. Utilisation de la composition alimentaire pour animaux de compagnie selon l'une quelconque des revendications précédentes qui est une composition alimentaire pour animaux de compagnie nutritionnellement équilibrée.

8. Utilisation de la composition alimentaire pour animaux de compagnie selon l'une quelconque des revendications 1 à 6 qui est un complément.
